# EUROPÄISCHE PATENTSCHRIFT

(11) **EP 1 743 000 B1**
(45) Veröffentlichungstag und Bekanntmachung des Hinweises auf die Patenterteilung: **30.07.2008**
(21) Anmeldenummer: 05733714.9
(22) Anmeldetag: 15.04.2005
(51) Int. Cl.: C09B 67/00, C09D 11/00, C01B 13/14, C01F 7/02, C04B 35/111, C09D 133/10

(54) **OBERFLÄCHENMODIFIZIERTE METALLOXIDE, VERFAHREN ZUR HERSTELLUNG UND DEREN VERWENDUNG IN KOSMETISCHEN ZUBEREITUNGEN**
SURFACE-MODIFIED METAL OXIDES, METHOD FOR PRODUCING THEM, AND THEIR USE IN COSMETIC PREPARATIONS
OXYDES METALLIQUES A SURFACE MODIFIEE, PROCEDE POUR LES PRODUIRE ET LEUR UTILISATION DANS DES PREPARATIONS COSMETIQUES

(30) Priorität: 27.04.2004 DE 102004020767
(43) Veröffentlichungstag der Anmeldung: 17.01.2007
(73) Patentinhaber: BASF SE, 67056 Ludwigshafen (DE)
(72) Erfinder: ANDRE, Valerie, 67063 Ludwigshafen (DE); RIEGER, Jens, 67063 Ludwigshafen (DE); BOUILLO, Nathalie, 76532 Baden-Baden (DE)
(86) Internationale Anmeldenummer: PCT/EP2005/004000
(87) Internationale Veröffentlichungsnummer: WO 2005/105930

(56) Entgegenhaltungen:
- US-A- 5 800 601
- US-A1- 2002 150 678
- US-B1- 6 710 091

## Beschreibung

Die vorliegende Erfindung betrifft oberflächenmodifizierte nanopartikuläre Metalloxide, Verfahren zu ihrer Herstellung und deren Verwendung als UV-Filter in kosmetischen Zubereitungen.

Metalloxide finden für vielfältige Zwecke Verwendung, so z.B. als Weißpigment, als Katalysator, als Bestandteil antibakterieller Hautschutzsalben und als Aktivator für die Kautschukvulkanisation. In kosmetischen Sonnenschutzmitteln findet man feinteiliges Zinkoxid oder Titandioxid als UV-absorbierende Pigmente.

Mit dem Begriff "Nanopartikel" bezeichnet man im Rahmen der vorliegenden Anmeldung Partikel mit einem mittleren Durchmesser von 5 bis 10000 nm, bestimmt mittels elektronenmikroskopischer Methoden.

Zinkoxidnanoteilchen mit Partikelgrößen unterhalb ca. 30 nm sind potentiell für den Einsatz als UV-Absorber in transparenten organisch-anorganischen Hybridmaterialien, Kunststoffen, Lacken und Beschichtungen geeignet. Daneben ist auch ein Einsatz zum Schutz UV-empfindlicher organischer Pigmente möglich.

Partikel, Partikelaggregate oder -agglomerate aus Zinkoxid, die größer als ca. 30 nm sind, führen zu Streulichteffekten und damit zu einer unerwünschten Abnahme an Transparenz im Bereich des sichtbaren Lichts. Deshalb ist die Redispergierbarkeit, also die Überführbarkeit der hergestellten Zinkoxidnanoteilchen in einen kolloiddispersen Zustand, eine wichtige Voraussetzung für die oben genannten Anwendungen.

Zinkoxidnanoteilchen mit Partikelgrößen unterhalb ca. 5 nm zeigen aufgrund des Grö-βenquantisierungseffektes eine Blauverschiebung der Absorptionskante (L. Brus, J. Phys. Chem. (1986), 90, 2555-2560) und sind daher für den Einsatz als UV-Absorber im UV-A-Bereich weniger geeignet.

Bekannt ist die Herstellung von Metalloxiden, beispielsweise von Zinkoxid durch trockene und nasse Verfahren. Die klassische Methode der Verbrennung von Zink, die als trockenes Verfahren bekannt ist (z.B. Gmelin Band 32, 8. Aufl., Ergänzungsband, S. 772 ff.), erzeugt aggregierte Partikel mit einer breiten Größenverteilung. Zwar ist es grundsätzlich möglich, durch Mahlverfahren Teilchengrößen im Submikrometerbereich herzustellen, doch aufgrund der zu geringen erzielbaren Scherkräfte sind aus solchen Pulvern Dispersionen mit mittleren Teilchengrößen im unteren Nanometerbereich nicht erzielbar. Besonders feinteiliges Zinkoxid wird vor allem naßchemisch durch Fällungsprozesse hergestellt. Die Fällung in wässriger Lösung liefert in der Regel hydroxidund/oder carbonathaltige Materialien, die thermisch zu Zinkoxid umgesetzt werden müssen. Die thermische Nachbehandlung wirkt sich dabei auf die Feinteiligkeit negativ aus, da die Partikel dabei Sinterprozessen unterworfen sind, die zur Bildung mikrometergroßer Aggregate führen, die durch Mahlung nur unvollständig auf die Primärpartikel heruntergebrochen werden können.

Nanopartikuläre Metalloxide können beispielsweise durch das Mikroemulsionsverfahren erhalten werden. Bei diesem Verfahren wird eine Lösung eines Metallalkoxids zu einer Wasser-in-Öl-Mikroemulsion getropft. In den inversen Micellen der Mikroemulsion, deren Größe im Nanometerbereich liegt, findet dann die Hydrolyse der Alkoxide zum nanopartikulären Metalloxid statt. Die Nachteile dieses Verfahrens liegen insbesondere darin, dass die Metallalkoxide teure Ausgangsstoffe darstellen, dass zusätzlich Emulgatoren verwendet werden müssen und dass die Herstellung der Emulsionen mit Tröpfchengrößen im Nanometerbereich einen aufwendigen Verfahrensschritt darstellt.

In der DE 199 07 704 wird ein nanopartikuläres über eine Fällungsreaktion hergestelltes Zinkoxid beschrieben. Hierbei wird das nanopartikuläre Zinkoxid ausgehend von einer Zinkacetatlösung über eine alkalische Fällung hergestellt. Das abzentrifugierte Zinkoxid kann durch Zugabe von Methylenchlorid zu einem Sol redispergiert werden. Die so hergestellten Zinkoxiddispersionen haben den Nachteil, dass sie aufgrund fehlender Oberflächenmodifizierung keine gute Langzeitstabilität besitzen.

In der WO 00/50503 werden Zinkoxidgele beschrieben, die nanopartikuläre Zinkoxidpartikel mit einem Partikeldurchmesser von ≤ 15 nm enthalten und die zu Solen redispergierbar sind. Hierbei werden die durch basische Hydrolyse einer Zinkverbindung in Alkohol oder in einem Alkohol/Wassergemisch hergestellten Fällungen durch Zugabe von Dichlormethan oder Chloroform redispergiert. Nachteilig ist hierbei, dass in Wasser oder in wässrigen Dispergierungsmitteln keine stabilen Dispersionen erhalten werden.

In der Veröffentlichung aus Chem. Mater. 2000, 12, 2268-74 "Synthesis and Characterization of Poly(vinylpyrrolidone)-Modified Zinc Oxide Nanoparticles" von Lin Guo and Shihe Yang werden Wurtzit Zinkoxidnanopartikel mit Polyvinylpyrrolidon oberflächenbeschichtet. Der Nachteil hierbei ist, dass mit Polyvinylpyrrolidon beschichtete Zinkoxidpartikel nicht in Wasser dispergierbar sind.

In der WO 93/21127 wird ein Verfahren zur Herstellung oberflächenmodifizierter nanopartikulärer keramischer Pulver beschrieben. Hierbei wird ein nanopartikuläres keramisches Pulver durch Aufbringen einer niedrigmolekülaren organischen Verbindung, beispielsweise Propionsäure, oberflächenmodifiziert. Dieses Verfahren kann nicht zur Oberflächenmodifizierung von Zinkoxid eingesetzt werden, da die Modifizierungsreaktionen in wässriger Lösung durchgeführt werden und Zinkoxid sich in wässrigen organischen Säuren auflöst. Daher läßt sich dieses Verfahren nicht zur Herstellung von Zinkoxiddispersionen anwenden; darüberhinaus ist Zinkoxid in dieser Anmeldung auch nicht als mögliches Ausgangsmaterial für nanopartikuläre keramische Pulver genannt.

In JP-A-04 164 814 wird ein Verfahren beschrieben, welches durch Fällung in wässrigem Medium bei erhöhter Temperatur auch ohne thermische Nachbehandlung zu feinteiligem ZnO führt. Als mittlere Teilchengröße wird, ohne Angabe des Agglomerationsgrades, 20 - 50 nm angegeben. Diese Partikel sind verhältnismäßig groß. Dies führt schon bei minimaler Agglomeration zu Streueffekten, die in Transparentanwendungen unerwünscht sind.

In JP-A-07 232 919 wird die Herstellung 5 bis 10000 nm großer ZnO-Partikel aus Zinkverbindungen durch Umsetzung mit organischen Säuren und anderen organischen Verbindungen wie Alkoholen bei erhöhter Temperatur beschrieben. Die Hydrolyse erfolgt hier so, dass die entstehenden Nebenprodukte (Ester der eingesetzten Säuren) abdestilliert werden können. Das Verfahren erlaubt die Herstellung von ZnO-Pulvem, die durch zuvor erfolgte Oberflächenmodifizierung redispergierbar sind. Allerdings ist es auf Basis der Offenbarung dieser Anmeldung nicht möglich, Partikel mit einem mittleren Durchmesser < 15 nm herzustellen. In den in der Anmeldung aufgeführten Beispielen ist dementsprechend als kleinster mittlerer Primärpartikeldurchmesser 15 nm genannt.

Mit Organosiliciumverbindungen hydrophobierte Metalloxide werden u.a. beschrieben in DE 33 14 741 A1, DE 36 42 794 A1 und EP 0 603 627 A1 sowie in WO 97/16156.

Diese mit Silikonverbindungen gecoateten Metalloxide, beispielsweise Zinkoxid oder Titandioxid haben den Nachteil, dass damit hergestellte Öl-in-Wasser- bzw. Wasser-in-Öl-Emulsionen nicht immer die nötige pH-Wert Stabilität aufweisen.

Ferner beobachtet man häufig Unverträglichkeiten von verschiedenen, mit Silikonverbindungen gecoateten Metalloxiden untereinander, was zu einer unerwünschten Aggregatbildungen und zu Flokkulationen der verschiedenen Partikel führen kann.

Der vorliegenden Erfindung lag daher die Aufgabe zugrunde, nanopartikuläre Metalloxide bereitzustellen, die die Herstellung stabiler nanopartikulärer Dispersionen in Wasser oder polaren organischen Lösemitteln sowie in kosmetischen Ölen erlauben. Eine irreversible Aggregation der Partikel soll nach Möglichkeit vermieden werden, damit ein aufwendiger Mahlprozeß vermieden werden kann.

Diese Aufgabe wurde gelöst durch oberflächenmodifizierte nanopartikuläre Metalloxide, wobei das Metall ausgewählt ist aus der Gruppe, bestehend aus Aluminium, Cer, Eisen, Titan, Zink und Zirkonium, dadurch gekennzeichnet, dass die Oberflächenmodifikation eine Beschichtung mit einem Copolymer P mit einem K-Wert von mindestens 7 bis 130 aus
A) 60 bis 99 mol%, bevorzugt 70 bis 95 mol%, besonders bevorzugt 75 bis 90 mol-% eines N-Vinyllactams, und
B) 1 bis 40 mol-% eines Monomeren, ausgewählt aus der Gruppe der
   b₁) C₈-C₃₀-Alkylester von monoethylenisch ungesättigten C₃-C₈-Carbonsäuren
   b₂) N-Alkyl- oder N,N-Dialkyl-substituierten Amide der Acrylsäure oder der Methacrylsäure mit C₈-C₁₈-Alkylresten, oder
   b₃) der Vinylester von aliphatischen C₈-C₃₀-Carbonsäuren, oder Gemischen dieser Monomeren
C) 0 bis 39 mol% weiterer copolymerisierbarer Monomere
umfasst.

Als N-Vinyllactame kommen beispielsweise N-Vinylcaprolactam, N-Vinylpyrrolidon oder Mischungen davon in Frage. Bevorzugtes N-Vinyllactam ist N-Vinylpyrrolidon.

Als Comonomere B) kommen mindestens 1, höchstens 40 mol-%, bevorzugt 5 bis 30 mol-%, besonders bevorzugt 10 bis 25 mol-% eines Monomeren ausgewählt aus einer der folgenden Gruppen b₁) bis b₃) oder Mischungen dieser Comonomeren in Betracht.

Geeignete Monomere b₁) sind C₈-C₃₀-Alkylester von monoethylenisch ungesättigten C₃-C₈-Carbonsäuren. Geeignete Carbonsäuren sind beispielsweise Acrylsäure, Methacrylsäure, Dimethacrylsäure, Ethacrylsäure, Maleinsäure, Citraconsäure, Methylenmalonsäure, Allylessigsäure, Vinylessigsäure, Crotonsäure, Fumarsäure, Mesaconsäure oder Itaconsäure, wobei Acrylsäure und/oder Methacrylsäure bevorzugt sind. Geeignete Alkylreste umfassen auch Cycloalkylreste. Bevorzugt werden Ester mit C₈-C₁₈-Alkylresten eingesetzt. Geeignete Monomere sind beispielsweise Octylacrylat, 2-Ethylhexylacrylat, 2-Ethylhexylmethacrylat, Nonylacrylat, Decylacrylat, Laurylacrylat, Myristylacrylat, Cetylacrylat, Stearylacrylat, Oleylacrylat, Behenylacrylat, Hexylmethacrylat, Octylmethacrylat, Nonylmethacrylat, Decylmethacrylat, Laurylmethacrylat, Myristyl-methacrylat, Cetylmethacrylat, Stearylmethacrylat, Oleyl-methacrylat, Behenylmethacrylat oder tert.-Butylcyclohexylacrylat.

Weiterhin eignen sich als Monomere b₂) auch N-alkyl- oder N,N-dialkylsubstituierte Carbonsäureamide der Acrylsäure oder der Methacrylsäure, wobei es sich bei den Alkylresten um C₈-C₁₈-Alkyl- oder Cycloalkylreste handelt, zum Beispiel N-Stearylacrylamid, N-Stearylmethacrylamid, N-Octylacrylamid, N,N-Dioctylacrylamid, N,N-Dioctylmethacrylamid, N-Cetylacrylamid, N-Cetylmethacrylamid, N-Dodecylacrylamid, N-Dodecylmethacrylamid, N-Myristylacrylamid, 2-Ethylhexylacrylamid. Im Falle der N,N-Dialkylamide sind C₈- und C₉-Alkylreste bevorzugt.

Als weitere Monomere B) eignen sich Vinylester aliphatischer Carbonsäuren (C₈-C₃₀-Carbonsäuren). Bevorzugt finden Vinylester von C₈-C₁₈-Carbonsäuren Verwendung, beispielsweise Vinylester der Octan-, Nonan-, Decan-, Undeca-, Laurin-, Tridecan-, Myristin-, Palmitin-, Stearin-, Arachin- oder Behensäure oder der Ölsäure.

Selbstverständlich können auch Mischungen aus zwei oder mehreren Carbonsäureestem, Carbonsäureamiden oder Vinylestern eingesetzt werden, solange die Summe der Anteile dieser Comonomere nicht 50 mol-% überschreitet.

Bevorzugte Comonomere B) sind C₈-C₁₈-Alkylester der Acrylsäure und Methacrylsäure, besonders bevorzugt C₈-C₁₂-Alkylester der Acrylsäure, beispielsweise Decylacrylat, Laurylacrylat, Myristylacrylat und Cetylacrylat.

Als weitere Comonomere C) können folgende copolymerisierbaren Monomere (oder auch Mischungen derselben) verwendet werden (0 bis 39 mol-%, bevorzugt 0 bis 20 mol-%, besonders bevorzugt 0 bis 10 mol-%).

Monoethylenisch ungesättigte Carbonsäuren mit 3 bis 8 C-Atomen wie Acrylsäure, Methacrylsäure, Dimethacrylsäure, Ethacrylsäure, Maleinsäure, Citraconsäure, Methylenmalonsäure, Allylessigsäure, Vinylessigsäure Crotonsäure, Fumarsäure, Mesaconsäure und Itaconsäure. Aus dieser Gruppe von Monomeren verwendet man vorzugsweise Acrylsäure, Methacrylsäure, Maleinsäure oder Mischungen der genannten Carbonsäuren. Die monoethylenisch ungesättigten Carbonsäuren können in Form der freien Säure und - soweit vorhanden - der Anhydride oder in partiell oder in vollständig neutralisierter Form bei der Copolymerisation eingesetzt werden. Um diese Monomeren zu neutralisieren, verwendet man vorzugsweise Alkalimetall- oder Erdalkalimetallbasen, Ammoniak oder Amine, z.B. Natronlauge, Kalilauge, Soda, Pottasche, Natriumhydrogencarbonat, Magnesiumoxid, Calciumhydroxid, Calciumoxid, gasförmiges oder wäßriges Ammoniak, Triethylamin, Ethanolamin, Diethanolamin, Triethanolamin, Morpholin, Diethylentriamin oder Tetraethylenpentamin.

Weitere geeignete Comonomere C) sind beispielsweise die C₁-C₄-Alkylester, Amide und Nitrile der oben angegebenen Carbonsäuren, z.B. Acrylsäuremethylester, Acrylsäureethylester, Methacrylsäure-methylester, Methacrylsäureethylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonämethylester, Malein-säuredimethylester, Maleinsäuremonoethylester, Maleinsäuredi-ethylester, Acryl-amid, Methacrylamid, N,N-Dimethylacrylarnid, N-tert.-butylacrylamid, Acrylnitril, Methacrylnitril, Dimethylaminoethylacrylat, Diethylaminoethylacrylat, Diethylaminoethylmethacrylat sowie die Salze der zuletzt genannten Monomeren mit Carbonsäuren oder Mineralsäuren sowie die quatemierten Produkte.

Außerdem eignen sich als andere copolymerisierbare Monomere C) Acrylamidoglycolsäure, Vinylsulfonsäure, Allylsulfonsäure, Methallylsulfonsäure, Styrolsulfonsäure, Acrylsäure-(3-sulfo-propyi)ester, Methacrylsäure-(3-sulfopropyl)ester und Acrylamidomethylpropansulfonsäure sowie Phosphonsäuregruppen enthaltende Monomere, wie Vinylphosphonsäure, Allylphosphonsäure und Acrylamidomethanpropanphosphonsäure.

Weitere geeignete copolymerisierbare Verbindungen C) sind, N-Vinylimidazol, N-Vinyl-2-methylimidazol, N-Vinyl-4-methylimidazol, Diallylammoniumchlorid, Vinylacetat und Vinylpropionat. Es können auch Mischungen der genannten Monomeren einzusetzen.

Bevorzugte Comonomere C) sind Alkali- oder Erdalkalisalze der Acrylsäure und Methacrylsäure, besonders bevorzugt Natriumacrylat und Natriummethacrylat.

Die Herstellung der Copolymerisate erfolgt nach bekannten Verfahren, z.B. der Lösungs-, Fällungs-, oder umgekehrten Suspensionspolymerisation unter Verwendung von Verbindungen, die unter den Polymerisationsbedingungen Radikale bilden.

Die Polymerisationstemperaturen liegen üblicherweise in dem Bereich von 30 bis 200, vorzugsweise 40 bis 110°C. Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, z.B. Natriumsulfit, Natriumbisulfit, Natriumformaldehydsulfoxilat und Hydrazin.

Die Copolymeren P besitzen K-Werte von mindestens 7 bis 130, vorzugsweise 10 bis 100, besonders bevorzugt 10 bis 20. Die K-Werte werden bestimmt nach H. Fikentscher, Cellulose-Chemie, Band 13, 58 bis 64 und 71 bis 74 (1932) in wässriger 0,1 M NaCI-Lösung bei 25°C, bei Polymerkonzentrationen, die je nach K-Wert-Bereich zwischen 0,1% und 5%, bevorzugt bei 1 % liegen.

Eine bevorzugte Ausführungsform der erfindungsgemäßen Metalloxide ist dadurch gekennzeichnet, dass die Metalloxidpartikel einen mittleren Primärpartikeldurchmesser von 5 bis 10000 nm, bevorzugt von 10 bis 200 nm, besonders bevorzugt von 10 bis 50 nm aufweisen, Partikeldurchmesser bestimmt mittels Raster- und Transmissionselektronenmikroskopie.

Als bevorzugte Metalloxide sind im Rahmen der vorliegenden Erfindung Titandioxid und Zinkoxid, besonders bevorzugt Zinkoxid zu nennen.

Der Erfindung liegt die Erkenntnis zugrunde, dass durch eine Oberflächenmodifikation von nanopartikulären Metalloxiden mit Copolymer P eine Langzeitstabilität von Dispersionen der oberflächenmodifizierten Metalloxide, insbesondere in kosmetischen Zubereitungen ohne unerwünschte pH-Wert Änderungen bei der Lagerung dieser Zubereitungen erreicht werden kann.

Ein weiterer Gegenstand der Erfindung ist ein Verfahren zur Herstellung eines oberflächenmodifizierten nanopartikulären Metalloxids, wobei das Metall ausgewählt ist aus der Gruppe, bestehend aus Aluminium, Cer, Eisen, Titan, Zink und Zirkonium, durch
a. Fällung des Metalloxids aus einer wässrigen Lösung eines seiner Metallsalze, wobei im Falle von Zinkoxid der pH-Wert bei der Fällung im Bereich von 7 bis 11 liegt
b. Abtrennung des ausgefällten Metalloxids aus der wässrigen Reaktionsmischung und
c. anschließende Trocknung des Metalloxids,
   dadurch gekennzeichnet, dass die Fällung des Metalloxids im Verfahrensschritt a. in Gegenwart eines Copolymeren P mit einem K-Wert von mindestens 7 bis 130 aus
   A) 60 bis 99 mol-% eines N-Vinyllactams, beispielsweise N-Vinylpyrrolidon, und/oder N-Vinylcaprolactam, bevorzugt N-Vinylpyrrolidon
   B) 1 bis 40 mol-% eines Monomeren, ausgewählt aus der Gruppe der
      b₁) C₈-C₃₀-Alkylester von monoethylenisch ungesättigten C₃-C₈-Carbonsäuren
      b₂) N-Alkyl- oder N,N-Dialkyl-substituierten Amide der Acrylsäure oder der Methacrylsäure mit C₈-C₁₈-Alkylresten, oder
      b₃) der Vinylester von aliphatischen C₈-C₃₀-Carbonsäuren, oder Gemischen dieser Monomeren.
   C) 0 bis 39 mol-% weiterer copolymerisierbarer Monomere
   erfolgt.

Bei den Metallsalzen im Verfahrensschritt a. kann es sich um Metallhalogenide, - acetate, -sulfate oder -nitrate handeln. Bevorzugte Metallsalze sind dabei Halogenide, beispielsweise Zink(II)chlorid oder Titantetrachlorid sowie Nitrate, beispielsweise Zink(II)nitrat, sowie Acetate, beispielsweise Zink(II)acetat.

Die Fällung des Metalloxids im Verfahrensschritt a. kann bei einer Temperatur im Bereich von 20°C bis 100°C, bevorzugt im Bereich von 25°C bis 40°C erfolgen.

Je nach verwendetem Metallsalz kann die Fällung bei einem pH-Wert im Bereich von 3 bis 13 durchgeführt werden. Im Falle von Zinkoxid liegt der pH-Wert bei der Fällung im Bereich von 7 bis 11.

Die Konzentration der Metallsalze liegt in der Regel im Bereich von 0,05 bis 1 mol/I, bevorzugt im Bereich von 0,1 bis 0,5 mol/l, besonders bevorzugt im Bereich von 0,2 bis 0,4 mol/l.

Die Fällzeit beträgt in der Regel 2 bis 8 Stunden, bevorzugt 3 bis 7 Stunden, besonders bevorzugt 4 bis 6 Stunden.

Bezüglich der Definition des im erfindungsgemäßen Verfahren als Oberflächenbeschichtung eingesetzten Copolymers P, sowohl in der allgemeinen als auch bevorzugten Ausführungsform, sei auf die eingangs erfolgte Beschreibung hingewiesen.

Gegenstand der vorliegenden Erfindung ist insbesondere ein Verfahren zur Herstellung von oberflächenmodifiziertem nanopartikulären Zinkoxid durch
a. Fällung des Zinkoxids aus einer wässrigen Lösung von Zink(II)chlorid, Zink(II)nitrat oder Zink(II)acetat bei einer Temperatur im Bereich von 25 bis 40°C und einem pH-Wert im Bereich von 7 bis 11 in Gegenwart eines Alkalimetallhydroxids,
b. Abtrennung des ausgefällten Zinkoxids aus der wässrigen Reaktionsmischung und
c. anschließende Trocknung,
dadurch gekennzeichnet, dass die Fällung des Zinkoxids im Verfahrensschritt a. in Gegenwart von Copolymer P erfolgt.

Die Fällung des Zinkoxids im Verfahrensschritt a. kann beispielsweise erfolgen durch Zudosierung einer wässrigen Lösung eines Gemisches aus Copolymer P und eines Alkalimetallhydroxids oder Ammoniumhydroxids, insbesondere NaOH zu der wässrigen Lösung von Zink(II)chlorid, Zink(II)nitrat oder Zink(II)acetat oder durch gleichzeitiges Zusdosieren jeweils einer wässrigen Lösung von Zink(II)chlorid, Zink(II)nitrat oder Zink(II)acetat und einer wässrigen Lösung eines Alkalimetallhydroxids oder Ammoniumhydroxids, insbesondere NaOH zu einer wässrigen Lösung des Coplolymers P.

Die Abtrennung des ausgefällten Metalloxids aus der wässrigen Reaktionsmischung kann in an sich bekannter Weise beispielsweise durch Filtration oder Zentrifugation erfolgen.

Der erhaltene Filterkuchen kann in an sich bekannter Weise getrocknet werden, beispielsweise im Trockenschrank bei Temperaturen zwischen 40 und 100°C, bevorzugt zwischen 50 und 70°C unter Normaldruck bis zur Gewichtskonstanz.

Ein weiterer Gegenstand der vorliegenden Erfindung ist ein kosmetisches Mittel, das ein erfindungsgemäß oberflächenbeschichtetes Zinkoxid oder eine Zinkoxiddispersion enthält.

Ein weiterer Gegenstand der vorliegenden Erfindung ist die Verwendung von oberflächenmodifiziertem Metalloxid, insbesondere Titandioxid oder Zinkoxid, die nach dem erfindungsgemäßen Verfahren hergestellt sind:
- zum UV-Schutz
- als antimikrobieller Wirkstoff

Gemäß einer bevorzugten Ausführungsform der vorliegenden Erfindung ist das oberflächenmodifizierte Metalloxid, insbesondere Titandioxid oder Zinkoxid in einem flüssigen Medium redispergierbar und bildet stabile Dispersionen. Dies ist besonders vorteilhaft, weil die aus dem erfindungsgemäßen Zinkoxid hergestellten Dispersionen vor der Weiterverarbeitung nicht erneut dispergiert werden müssen, sondern direkt verarbeitet werden können.

Nach einer bevorzugten Ausführungsform der vorliegenden Erfindung ist das oberflächenmodifizierte Metalloxid in polaren organischen Lösemitteln redispergierbar und bildet stabile Dispersionen. Dies ist besonders vorteilhaft, da hierdurch eine gleichmäßige Einarbeitung beispielsweise in Kunststoffe oder Folien möglich ist.

Nach einer weiteren bevorzugten Ausführungsform der vorliegenden Erfindung ist das oberflächenmodifizierte Metalloxid in Wasser redispergierbar und bildet dort stabile Dispersionen. Dies ist besonders vorteilhaft, da sich hierdurch die Möglichkeit eröffnet, das erfindungsgemäße Material beispielsweise in kosmetischen Rezepturen einzusetzen, wobei der Verzicht auf organische Lösemittel einen großen Vorteil darstellt. Denkbar sind auch Mischungen von Wasser und polaren organischen Lösemitteln.

Gemäß einer bevorzugten Ausführungsform der vorliegenden Erfindung besitzen die oberflächenmodifizierten Metalloxidpartikel einen Durchmesser von 10 bis 200 nm. Dies ist besonders vorteilhaft, da innerhalb dieser Größenverteilung eine gute Redispergierbarkeit gewährleistet ist.

Gemäß einer besonders bevorzugten Ausführungsform der vorliegenden Erfindung weisen die Metalloxidnanopartikel einen Durchmesser von 10 bis 50 nm auf. Dieser Größenbereich ist besonders vorteilhaft, da nach Redispergierung von solchen Zinkoxidnanopartikeln die entstehenden Dispersionen transparent sind und somit beispielsweise bei Zugabe zu kosmetischen Rezepturen die Farbgebung nicht beeinflussen. Darüber hinaus ergibt sich hierdurch auch die Möglichkeit zum Einsatz in transparenten Folien.

Wenn die Metalloxide, insbesondere Titandioxid oder Zinkoxid als UV-Absorber eingesetzt werden sollen, ist es ratsam, Partikel mit einem Durchmesser von mehr als 5 nm einzusetzen, da unterhalb dieser Grenze eine Verschiebung der Absorptionskante in den kurzwelligen Bereich erfolgt (L. Brus, J. Phys., Chem. (1986), 90, 2555 - 2560).

Ein weiterer Gegenstand der vorliegenden Erfindung ist ein kosmetisches Mittel, das ein erfindungsgemäß oberflächenmodifiziertes Metalloxid, insbesondere Titandioxid und/oder Zinkoxid enthält. Dies ist besonders vorteilhaft, da aufgrund der feinen Verteilung der Metalloxidpartikel, insbesondere der Zinkoxidpartikel diese ihre hautberuhigende Wirkung effektiver entfalten können.

Ein weiterer Vorteil liegt darin, dass beim Auftragen auf z.B. die Haut aufgrund der geringen Partikelgröße kein Reibeeffekt auftritt, sondern ein sanftes Auftragen möglich ist, was ein angenehmes Hautgefühl hervorruft.

Nach einer weiteren Ausführungsform des kosmetischen Mittels dient dieses der Pflege oder dem Schutz der Haut insbesondere zum Sonnenschutz bzw. zur Pflege bei Sonnenlichtexposition und liegt in Form einer Emulsion, einer Dispersion, einer Suspension, einer wässrigen Tensidzubereitung, einer Milch, einer Lotion, einer Creme, eines Balsams, einer Salbe, eines Gels, eines Granulats, eines Puders, eines Stiftpräparates, wie z.B. eines Lippenstifts, eines Schaums, eines Aerosols oder eines Sprays vor. Solche Formulierungen sind gut geeignet für topische Zubereitungen. Als Emulsionen kommen Öl-in-Wasser-Emulsionen und Wasser-in-Öl-Emulsionen oder Mikroemulsionen in Frage. Dies ist besonders vorteilhaft, weil durch den Einsatz in Sonnenschutzmittel die UV-absorbierende und die hautberuhigende Wirkung beispielsweise des Zinkoxids gleichzeitig genutzt werden können. Darüber hinaus eignen sich die erfindungsgemäß oberflächenmodifizierten Metalloxide sehr gut zum Einsatz in Sonnenschutzmittel, da die Partikel in einer Größe herstellbar sind, die sie für das menschliche Auge tränparent erscheinen lassen. Dadurch entsteht bei der Anwendung kein weißer Schleier auf der Haut.

Ein weiterer Vorteil ist die Tatsache, dass es sich insbesondere bei Zinkoxid um einen UV-Breitbandfilter handelt, dessen UV-Absorptionsverhalten es erlaubt, ein Sonnenschutzmittel zu schaffen, dass keine weiteren chemischen UV-Filtersubstanzen mehr benötigt. Dadurch kann die Gefahr von Haut-Irritationen oder allergischen Reaktionen durch Zersetzungsprodukte chemischer Filter oder durch diese Substanzen selbst vermieden werden, was die allgemeine Verträglichkeit eines derart gestalteten Sonnenschutzmittels stark erhöht. Im Regelfall wird das kosmetische Mittel zur topischen Applikation auf der Haut verwendet. Unter topischen Zubereitungen sind dabei solche Zubereitungen zu verstehen, die dazu geeignet sind, die Wirkstoffe in feiner Verteilung und bevorzugt in einer durch die Haut resorbierbaren Form auf die Haut aufzubringen. Hierfür eignen sich z.B. wässrige und wässrig-alkoholische Lösungen, Sprays, Schäume, Schaumaerosole, Salben, wässrige Gele, Emulsionen vom O/W- oder W/O-Typ, . Mikroemulsionen oder kosmetische Stiftpräparate.

Nach einer bevorzugten Ausführungsform des erfindungsgemäßen kosmetischen Mittels enthält das Mittel einen Träger. Bevorzugt als Träger ist Wasser, ein Gas, eine Wasser-basierte Flüssigkeit, ein Öl, ein Gel, eine Emulsion oder Mikroemulsion, eine Dispersion oder eine Mischung davon. Die genannten Träger zeigen eine gute Hautverträglichkeit. Besonders vorteilhaft für topische Zubereitungen sind wässrige Gele, Emulsionen oder Mikroemulsionen.

Als Emulgatoren können nichtionogene Tenside, zwitterionische Tenside, ampholytische Tenside oder anionische Emulgatoren verwendet werden. Die Emulgatoren können in der erfindungsgemäßen Zusammensetzung in Mengen von 0,1 bis 10, vorzugsweise 1 bis 5, Gew.-%, bezogen auf die Zusammensetzung, enthalten sein.

Als nichtionogenes Tensid kann beispielsweise ein Tensid aus mindestens einer der folgenden Gruppen verwendet werden:
Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
C_{12/18}-Fettsäuremono- und -diester von Anlagerungsprodukten von 1 bis 30 Mol Ethylenoxid an Glycerin;
Glycerinmono- und -diester und Sorbitanmono- und -diester von gesättigten und ungesättigten Fettsäuren mit 6 bis 22 Kohlenstoffatomen und deren Ethylenoxidanlagerungspfodukte;
Alkylmono- und -oligoglycoside mit 8 bis 22 Kohlenstoffatomen im Alkylrest und deren ethoxylierte Analoga;
Anlagerungsprodukte von 15 bis 60 Mol Ethylenoxid an Ricinusöl und/oder gehärtetes Ricinusöl;
Polyol- und insbesondere Polyglycerinester, wie z.B. Polyglycerinpolyricinoleat, Polygl ycerinpoly-12-hydroxystearat oder Polyglycerindimerat. Ebenfalls geeignet sind Gemische von Verbindungen aus mehreren dieser Substanzklassen;
Anlagerungsprodukte von 2 bis 15 Mol Ethylenoxid an Ricinusöl und/oder gehärtetes Ricinusöl;
Partialester auf Basis linearer, verzweigter, ungesättigter bzw. gesättigter C_{6/22} - Fettsäuren, Ricinolsäure sowie 12-Hydroxystearinsäure und Glycerin, Polyglycerin, Pentaerythrit, Dipenta-erythrit, Zuckeralkohole (z. B. Sorbit), Alkylglucoside (z.B. Methylglucosid, Butylglucosid, Lauryl-glucosid) sowie Polyglucoside (z.B. Cellulose);
Mono-, Di- und Trialkylphosphate sowie Mono-, Di- und/oder Tri-PEG-alkylphosphate und deren Salze;
Wollwachsalkohole;
Polysiloxan-Polyalkyl-Polyether-Copolymere bzw. entsprechende Derivate;
Mischester aus Pentaerythrit, Fettsäuren, Citronensäure und Fettalkohol gemäß DE PS 1165574 und/oder Mischester von Fettsäuren mit 6 bis 22 Kohlenstoffatomen, Methylglucose und Polyolen, vorzugsweise Glycerin oder Polyglycerin sowie
Polyalkylenglycole;
Betaine.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- oder eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethyl-ammoniumglycinat, N-Acylam'ino-propyl-N,N dimethylammoniumglycinate, beispielsweise das Kokosacylaminopropyldimethylammoniuni-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethyl-carboxymethylglycinat. Besonders bevorzugt ist das unter der CTFA Bezeichnung Cocamidopropyl Betaine bekannte Fettsäureamid-Derivat.

Ebenfalls geeignete Emulgatoren sind ampholytische Tenside. Unter ampholytischen Tensiden werden solche oberflächenaktiven Verbindungen verstanden, die außer einer C_{8.18}-Alk-yl- oder-Acylgruppe im Molekül mindestens eine freie Aminogruppe und mindestens eine -COOH- oder -S0₃H-Gruppe enthalten und zur Ausbildung innerer Salze befähigt sind. Beispiele für geeignete ampholytische Tenside sind N-Alkylglycine, N-Alkylpropionsäuren , N-Alkylamino-buttersäuren , N Alkyliminodipropionsäuren, N-Hydroxyethyl-N-alkylämido-propylglycine, N-Alkyltaurine, N Alkylsarcosine, 2-Alkylaminopropionsäuren und Alkylaminoessigsäuren mit jeweils etwa 8 bis 18 C-Atomen in der Alkylgruppe.

Besonders bevorzugte ampholytische Tenside sind das N-Kokosalkyl-aminopropionat , Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quatemierte Difettsäuretriethanolaminester-Salze, besonders bevorzugt sind. Des weiteren können als anionische Emulgatoren Alkylethersulfate, Monoglyceridsulfate, Fettsäuresulfate, Sulfosuccinate und/oder Ethercarbonsäuren eingesetzt werden.

Als Ölkörper kommen Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettaikohoien, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-, Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv^{®} TN), Dialkylether, Ringöffnungsprodukte von epoxidierten Fettsäureestern mit Polyolen, Siliconöle und/oder aliphatische bzw. naphthenische Kohlenwasserstoffe in Betracht. Als Ölkörper können ferner auch Siliconverbindungen eingesetzt werden, beispielsweise Dimethylpolysiloxane, Methylphenylpolysiloxane, methylpolysiloxane, Methylphenylpolysiloxane, cyclische Silicone sowie amino-, fettsäure-, alkohol-, polyether-, epoxy-, fluor-, alkyl- und/oder glykosidmodifizierte Siliconverbindüngen, die bei Raumtemperatur sowohl flüssig als auch harzförmig vorliegen können. Die Ölkörper können in den erfindungsgemäßen Mitteln in Mengen von 1 bis 90, vorzugsweise 5 bis 80, und insbesondere 10 bis 50 Gew.-%, bezogen auf die Zusammensetzung enthalten sein.

Nach einer besonders bevorzugten Ausführungsform enthält die erfindungsgemäße Zusammensetzung weitere UV-Lichtschutzfilter in Form löslicher Verbindungen oder anderer Pigmente.

Obwohl es wie bereits weiter oben beschrieben möglich ist, mit Hilfe der erfindungsgemäßen Zinkoxidpartikel ein Sonnenschutzmittel zu schaffen, das gute UV Absorptionseigenschaften ohne weitere UV-Filtersubstanzen erreicht, kann es im Einzelfall gewünscht sein, dem kosmetischen Mittel bzw. dem Sonnenschutzmittel weitere UV-Filtersubstanzen zuzusetzen. Dies kann z.B. dann erforderlich sein, wenn ein besonderer Schwerpunkt bei der Filterleistung gelegt werden soll. Der erfindungsgemäßen Zusammensetzung können ein oder mehrere weitere UV-Lichtschutzfilter zugesetzt werden.

Im Falle der löslichen Verbindungen sind unter UV-Lichtschutzfiltern organische Substanzen zu verstehen, die in der Lage sind, ultraviolette Strahlen zu absorbieren und die aufgenommene Energie in Form längerwelliger Strahlung, z.B. Wärme, wieder abzugeben. Die organischen Substanzen können öllöslich oder wasserlöslich sein.

Als öllösliche UV-B-Filter können z.B. folgende Substanzen verwendet werden:
3-Benzylidencampher und dessen Derivate, z.B. 3-(4-Methylbenzyliden)campher;
4-Aminobenzoesäurederivate, vorzugsweise 4-(Dimethylamino)benzoesäure-2-ethylhexylester, 4-( Dimethylamino)benzoesäure-2-octylester und 4-(Dimethylamino)-benzoesäureamylester;
Ester der Zimtsäure, vorzugsweise 4-Methoxyzimtsäure-2-ethylhexylester, 4 Methoxyzimtsäurepropylester, 4-Methoxyzimtsäureisoamylester, 4 Methoxyzimtsäureisopentylester, 2-Cyano-3-phenyl-zimtsäure-2-ethylhexylester (Octocrylene);
Ester der Salicylsäure, vorzugsweise Salicylsäure-2-ethylhexylester, Salicylsäure-4 isopropylbenzylester, Salicylsäurehomomenthylester;
Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
Ester der Benzalmalonsäure, vorzugsweise 4-Methoxybenzmalonsäuredi-2-ethylheicylester;
Triazinderivate, wie z.B. 2,4,6-Trianilino-(p-carbo-2'-ethyl-1'-hexyloxy)-1,3,5-triazin (Octyltriazone) und Dioctyl Butamido Triazon (Uvasorb^{®} HEB).
Propan-1,3-dione, wie z.B. 1 -(4-tert. Butylphenyl)-3-(4'-methoxyphenyl)propan-1,3-dion.

Als wasserlösliche Substanzen kommen in Frage:
2-Phenylbenzimidazol-5-sulfonsäure und deren Alkali-, Erdalkali-, Ammonium-, Alkylammonium-, Alkanolammonium- und Glucammoniumsalze;
Sulfonsäurederivate von Benzophenonen , vorzugsweise 2-Hydroxy-4-methoxybenzophenon-5-sulfonsäure und ihre Salze;
Sulfonsäurederivate des 3-Benzylidencamphers, wie z.B. 4-(2-Oxo-3-bomylidenmethyl)benzolsulfonsäure und 2-Methyl-5-(2-oxo-3-bornyliden)sulfonsäure und deren Salze.

Besonders bevorzugt ist die Verwendung von Estern der Zimtsäure, vorzugsweise 4-Methoxyzimtsäure-2-ethylhexylester, 4-Methoxyzimtsäureisopentylester, 2-Cyano-3-phenyl-zimtsäure-2-ethylhexylester (Octocrylene).

Des weiteren ist die Verwendung von Derivaten des Benzophenons, insbesondere 2-Hydroxy-4-methoxybenzophenon, 2-Hydroxy-4-methoxy-4'-methylbenzophenon, 2,2'-Dihydroxy-4-methoxybenzophenon sowie der Einsatz von Propan-1,3-dionen, wie z.B. 1-(4-tert. Butylphenyl)-3-(4-'methoxyphenyl)propan-1,3-dion bevorzugt.

Als typische UV-A-Filter kommen in Frage:
Derivate des Benzoylmethans, wie beispielsweise 1-(4'-tert.Butylphenyl)-3-(4'-methoxyphenyl)propan-1,3-dion, 4-tert.-Butyl-4'-methoxydibenzoylmethan oder 1-Phenyl-3-(4'-isopropylphenyl)-propan-1,3-dion;
Amino-hydroxy-substituierte Derivate von Benzophenonen wie z.B. N,N-Diethylaminohydroxybenzoyl-n-hexylbenzoat.

Die UV-A und UV-B-Filter können selbstverständlich auch in Mischungen eingesetzt werden.

Als weitere Lichtschutzfiiter können aber auch andere unlösliche Pigmente, z.B. feindisperse Metalloxide bzw. Salze wie beispielsweise Titandioxid, Eisenoxid, Aluminiumoxid, Ceroxid, Zirkoniumoxid, Silicate (Talk), Bariumsulfat und Zinkstearat verwendet werden. Die Partikel sollten dabei einen mittleren Durchmesser von weniger als 100 nm, vorzugsweise zwischen 5 und 50 nm und insbesondere zwischen 15 und 30 nm aufweisen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Der Gesamtanteil der Lichtschutzmittel in dem erfindungsgemäßen Sonnenschutzmittel liegt üblicherweise bei 1 bis 20, vorzugsweise 5 bis 15 Gew.-%. Die erfindungsgemäße Zusammensetzung als solche kann 1 bis 95 vorzugsweise 5 bis 80, und insbesondere 10 bis 60 Gew.-% Wasser enthalten.

Nach einer besonders bevorzugten Ausführungsform enthält die erfindungsgemäße kosmetische Zusammensetzung ferner pflegende Substanzen, weitere kosmetische Wirkstoffe und/oder Hilfs- und Zusatzstoffe.

Als weitere kosmetische Wirkstoffe werden insbesondere Hautfeuchthaltemittel, antimikrobielle Stoffe und/oder deodorierende oder schweißhemmende Stoffe eingesetzt. Dies hat den Vorteil, dass sich weitere gewünschte Effekte erzielen lassen, die zur Pflege oder Behandlung der Haut beitragen oder beispielsweise das Wohlempfinden des Anwenders der kosmetischen Zusammensetzung bei der Verwendung dieser Zusammensetzung steigern.

So können in der kosmetischen Zusammensetzung neben dem Träger, dem oberflächenmodifizierten Zinkoxid, Wasser und physiologisch geeigneten Lösemitteln unter anderem auch pflegende Bestandteile, wie z.B. Öle, Wachse, Fette, rückfettende Substanzen, Verdickungsmittel, Emulgatoren und Duftstoffe enthalten sein. Ein hoher Anteil an pflegenden Substanzen ist insbesondere zur topischen prophylaktischen oder kosmetischen Behandlung der Haut vorteilhaft.

Besonders vorteilhaft ist es, wenn die Zusammensetzung neben den in vielen Fällen ebenfalls Pflegewirkung aufweisenden tierischen und pflanzlichen Fetten und Ölen noch weitere Pflegekomponenten enthält. Die Gruppe der pflegenden Wirkstoffe, die zum Einsatz kommen können, umfasst z.B.: Fettalkohole mit 8-22 C-Atomen, insbesondere Fettalkohole von natürlichen Fettsäuren; tierische und pflanzliche Proteinhydrolysate, insbesondere Elastin-, Kollagen-, Keratin-, Milcheiweiß-, Sojaprotein-, Seidenprotein-, Haferprotein-, Erbsenprotein-, Mandelprotein- und Weizenproteinhydrolysate; Vitamine und Vitaminvorstufen, insbesondere die der Vitamin-Gruppen A und B; Mono-, Di- und Oligosaccharide; Pflanzenextrakte; Honigextrakte; Ceramide; Phospholipide; Vaseline, Paraffin- und Silikonöle; Fettsäure- und Fettalkoholester, insbesondere die Monoester der Fettsäuren mit Alkoholen mit 3 - 24 C-Atomen. Zu den in der erfindungsgemäßen Zusammensetzung bevorzugt einzusetzenden Vitaminen, Provitaminen oder Vitaminvorstufen gehören unter anderem:
Vitamine, Provitamine und Vitaminvorstufen aus den Gruppen A, C, E und F, insbesondere 3,4-Didehydroretinol, β-Carotin (Provitamin des Vitamin A), Ascorbinsäure (Vitamin C), sowie die Palmitinsäureester, Glucoside oder Phosphate der Ascorbinsäure, Tocopherole, insbesondere α-Tocopherol sowie seine Ester, z.B. das Acetat, das Nicotinat, das Phosphat und das Succinat; weiterhin Vitamin F, worunter essentielle Fettsäuren, besonders Linolsäure, Linolensäure und Arachidonsäure, verstanden werden;

Vitamin A und seine Derivate und Provitamine zeigen vorteilhafterweise einen besonderen hautglättenden Effekt.

Zu den in der erfindungsgemäßen Zusammensetzung bevorzugt einzusetzenden Vitaminen, Provitaminen oder Vitaminvorstufen der Vitamin B-Gruppe oder deren Derivaten sowie den Derivaten von 2-Furanon gehören unter anderem:

Vitamin B₁ , Trivialname Thiamin, chemische Bezeichnung 3-[(4'-Amino-2'-methyl-5'-pyrimidinyl) methyo-5-(2-hydroxyethyl)-4-methylthiazoliumchlorid. Bevorzugt wird Thiaminhydrochlorid in Mengen von 0,05 bis 1 Gew.-%, bezogen auf das gesamte Mittel, eingesetzt.

Vitamin B₂, Trivialname Riboflavin, chemische Bezeichung 7,8-Dimethyl-10-(1-D-ribityl)-benzo[g]pteridin-2,4(3H,10H)-dion. In freier Form kommt Riboflavin z. B. in Molke vor, andere Riboflavin-Derivate lassen sich aus Bakterien und Hefen isolieren. Ein erfindungsgemäß ebenfalls geeignetes Stereoisomeres des Riboflavin ist das aus Fischmehl oder Leber isolierbare Lyxoflavin, das statt des D-Ribityl einen D-Arabityl-Rest trägt. Bevorzugt werden Riboflavin oder seine Derivate in Mengen von 0,05 bis 1 Gew.-%, bezogen auf das gesamte Mittel, eingesetzt.

Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.

Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können zusätzlich zu Pantothensäure oder Panthenol auch Derivate des 2-Furanon eingesetzt werden. Besonders bevorzugte Derivate sind die auch im Handel erhältlichen Substanzen Dihydro-3 hydroxy-4,4-dimethyl-2(3H)-furanon mit dem Trivialnamen Pantolacton (Merck), 4 Hydroxymethyl-y-butyrolacton (Merck), 3,3-Dimethyl-2-hydroxy-y-butyrolacton (Aldrich) und 2,5- Dihydro-5-methoxy-2-furanon (Merck), wobei ausdrücklich alle Stereoisomeren eingeschlossen sind.

Vorteilhafterweise verleihen diese Verbindungen der erfindungsgemäßen kosmetischen Zusammensetzung feuchtigkeitsspendende sowie hautberuhigende Eigenschaften.

Die genannten Verbindungen des Vitamin B₅-Typs sowie die 2- Furanonderivate sind in den erfindungsgemäßen Mitteln bevorzugt in einer Gesamtmenge von 0,05 bis 10 Gew.-%, bezogen auf das gesamte Mittel, enthalten. Gesamtmengen von 0,1 bis 5 Gew.-% sind besonders bevorzugt.

Vitamin B₆, wobei man hierunter keine einheitliche Substanz, sondern die unter den Trivialnamen Pyridoxin, Pyridoxamin und Pyridoxal bekannten Derivate des 5 Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.

Vitamin B₇ (Biotin), auch als Vitamin H oder "Hautvitamin" bezeichnet. Bei Biotin handelt es sich um (3aS,4S, 6aR)-2-Oxohexahydrothienol[3,4-d]imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Panthenol, Pantolacton, Nicotinsäureamid sowie Biotin sind erfindungsgemäß ganz besonders bevorzugt.

Unter Hilfs- und Zusatzstoffen sind Stoffe zu verstehen, die zur Verbesserung der ästhetischen, anwendungstechnischen und/oder kosmetischen Eigenschaften geeignet sind, wie z.6. Co-Emulgatoren, organische Lösemittel, Überfettungsmittel, Stabilisatoren, Antioxidationsmittel, Wachse oder Fette, Konsistenzgeber, Verdickungsmittel, Bräunungsmittel, Vitamine, kationische Polymere, biogene Wirkstoffe, Konservierungsmittel, Hydrotope, Solubilisatoren, Farb- und Duftstoffe.

Beispielsweise können folgende Hilfs- und Zusatzstoffe verwendet werden:
- Allantoin,
- AloeVera,
- Bisabolol,
- Ceramide und Pseudoceramide,

Antioxidationsmittel verbessern vorteilhafterweise die Stabilität der erfindungsgemäßen Zusammensetzungen. Antioxidantien sind beispielsweise Aminosäuren (z.B. Glycin; Histidin, Tyrosin, Tryptophan) und deren Derivate, Imidazol und Imidazolderivate (z.B. Urocaninsäure), Peptide wie z.B. D,L-Camosin, D-Carnosin, L-Carnosin und deren Derivate (z. B. Anserin), Carotinoide, Carotine (z. 6. α-Carotin, ß-Carotin, Lycopin) und deren Derivate, Liponsäure und deren Derivate (z. B. Dihydroliponsäure), Aurothioglucose, Propylthiouracil und weitere Thioverbindungen (z.B. Thioglycerin, Thiosorbitol, Thioglycolsäure, Thi redoxin, Glutathion, Cystein, Cystin, Cystamin und deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl- und Glycerylester) sowie deren Salze, Dilauryl- thiodipropionat, Distearylthiodipropionat, Thiodipropionsäure und deren Derivate (Ester, Ether, Peptide, Lipide, Nukleotide, N kleoside und Salze) sowie Sulfoximinverbindungen (z. 6. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis pmol/kg), ferner Metallchelatoren (z.B. α-Hydroxyfettsäuren, EDTA, EGTA, Phytinsäure, Lactoferrin), α-Hydroxysäuren (z. B. Zitronensäure, Milchsäure, Äpfelsäure), Huminsäuren, Gallensäure, Gallenextrakte, Gallussäureester (z. B. Propyl-, Octyl- und Dodecylgallat), Flavonoide, Catechine, Bilirubin, Biliverdin und deren Derivate, ungesättigte Fettsäuren und deren Derivate (z.B. γ-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat, -stearat, -dipalmitat, acetat, Mg-Ascorbylphosphate, Natrium- und Magnesiumascorbat, Dinatriumascorbylphosphat und -sulfat, Kaliumascorbyltocopherylphosphat, Chitosanascorbat), Isoascorbinsäure und deren Derivate, Tocopherole und deren Derivate (z.B. Tocopherylacetat, -linoleat, -oleat und -succinat, Tocophereth-5, Tocophereth-10, Tocophereth-12, Tocophereth-18, Tocophereth-50, Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat, Zimtsäure und deren Derivate (z. B. Ferulasäure, Ethylferulat, Kaffeesäure), Kojisäure, Chitosanglycolat und - salicylat, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und Zink- Derivate (z. B. ZnO, ZnS04), Selen und Selen-Derivate (z. B. Selenmethionin), Stilbene und Stilben-Derivate (z. B. Stilbenoxid, trans-Stilbenoxid).

Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte (z.B. Teebaumöl, Rosmarinextrakt und Rosmarinsäure), die diese Antioxidantien enthalten, eingesetzt werden. Als lipophile, öllösliche Antioxidantien aus dieser Gruppe sind Tocopherol und dessen Derivate, Gallussäureester, Flavonoide und Carotinoide sowie Butylhydroxytoluollanisol bevorzugt. Als wasserlösliche Antioxidantien sind Aminosäuren, z. B. Tyrosin und Cystein und deren Derivate sowie Gerbstoffe, insbesondere solche pflanzlichen Ursprungs bevorzugt. Die Gesamtmenge der Antioxidantien in den erfindungsgemäßen kosmetischen Zusammensetzungen beträgt 0,001 - 20 Gew.-%, vorzugsweise 0,05 - 10 Gew.-%, insbesondere 0,1 - 5 Gew.-% und ganz besonders bevorzugt 0,1 bis 2 Gew.-%.

Triterpene, insbesondere Triterpensäuren wie Ursolsäure, Rosmarinsäure, Betulinsäure, Boswelliasäure und Bryonolsäure,
Monomere Catechine, besonders Catechin und Epicatechin, Leukoanthocyanidine, Catechinpolymere (Catechin-Gerbstoffe) sowie Gallotannine,
Verdickungsmittel, z. B. Gelatine, Pflanzengumme wie Agar-Agar, Guar-Gum, Alginate, Xanthan-Gum , Gummiarabicum , Karaya-Gummi oder Johannisbrotkernmehl, natürliche und synthetische Tone und Schichtsilikate, z. B. Bentonit, Hectorit, Montmorillonit oder Laponite^{®}, vollsynthetische Hydrokolloide wie z.B. Polyvinylalkohol, und außerdem Ca-, Mg- oder Zn-Seifen von Fettsäuren,
Pflanzenglycoside ,
Strukturanten wie Maleinsäure und Milchsäure,
Dimethylisosorbid,
Alpha-, beta- sowie gamma-Cyclodextrine, insbesondere zur Stabilisierung von Retinol,
Lösemittel, Quell- und Penetrationsstoffe wie Ethanol, Isopropanot, Ethylenglykol, Propylenglykol, Propylenglykolmonoethylether, Glycerin und Diethylenglykol, Carbonate, Hydrogencarbonate, Guanidine, Harnstoffe sowie primäre, sekundäre und tertiäre Phosphate
Parfümöle, Pigmente sowie Farbstoffe zum Anfärben des Mittels,
Substanzen zur Einstellung des pH-Wertes, z.B. α- und β-Hydroxycarbonsäuren,
Komplexbildner wie EDTA, NTA, ß-Alanindiessigsäure und Phosphorsäuren,
Trübungsmittel wie Latex, Styrol/PVP- und Styrol/Acrylamid-Copolymere,
Perlglanzmittel wie Ethylenglykolmono- und -distearat sowie PEG-3-distearat,
Treibmittel wie Propan-Butan-Gemische, N₂0, Dimethylether, CO₂ und Luft.

Die Zugabe von Allantoin, Bisabolol und/oder Aloe Vera auch in Form von Extrakten zu den erfindungsgemäßen kosmetischen Zusammensetzungen verbessert die weiterhin die hautberuhigenden, feuchtigkeitsspendenden und hautpflegende Eigenschaften der Formulierungen und ist daher besonders bevorzugt.

Als weitere Inhaltsstoffe kann die erfindungsgemäße kosmetische Zusammensetzung in untergeordneten Mengen weitere, mit den anderen Inhaltsstoffen kompatible Tenside enthalten.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Fettsäureamid-(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N- Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate.

Sofern die anionischen Tenside Polyglycoletherketten enthalten, können diese eine konventionelle, vorzugsweise jedoch eine eingeengte Homologenverteilung aufweisen.

Typische Beispiele für nichtionische Tenside sind Fettalkoholpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkyiglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Typische Beispiele für kationische Tenside sind quartäre Ammoniumverbindungen und Esterquats, insbesondere quaternierte Fettsäuretrialkanolaminestersalze.

Typische Beispiele für amphotere bzw. zwitterionische Tenside sind Alkylbetaine, Alkylamidobetaine, Aminopropionate, Aminoglycinate, Imidazoliniumbetaine und Sulfobetaine.

Nach einer weiteren besonders bevorzugten Ausführungsform wird das erfindungsgemäße kosmetische Mittel als Sonnenschutzmittel verwendet. Die daraus resultierenden Vorteile wurden bereits eingehend erläutert.

Der Einsatz der erfindungsgemäßen Zinkoxiddispersionen ist insbesondere ebenfalls möglich in Haarkosmetika wie Shampoos, Conditioner, Spülungen, Haarwässer, Haargel, Haarspray etc.. Insbesondere leave-on Produkte, die nach erfolgter Applikation auf dem Haar bzw. der Kopfhaut verbleiben sind besonders gut geeignet. Das so auf die Kopfhaut und das Haar aufgetragene Zinkoxid kann somit auch dort als UV Schutzmittel wirken bzw. auf der Kopfhaut seine hautberuhigende Wirkung entfalten.

Nach einer bevorzugten Ausführungsform des erfindungsgemäßen kosmetischen Mittels wird das kosmetische Mittel auf die Oberfläche des zu behandelnden bzw. zu schützenden Körpers also topisch aufgetragen. Diese Applikationsform ist besonders vorteilhaft, da sie einfach zu handhaben ist, so dass Fehldosierungen weitestgehend ausgeschlossen sind. Ferner lässt sich ein zusätzlicher pflegender Effekt für die Haut erreichen. Falls nur einzelne Körperteile der Sonnenstrahlung ausgesetzt werden, kann das Sonnenschutzmittel außerdem nur gezielt auf diese Körperteile aufgetragen werden.

Ein weiterer Gegenstand der vorliegenden Erfindung ist die Verwendung der erfindungsgemäß oberflächenmodifizierten Metalloxide zum UV Schutz. Dies ist besonders vorteilhaft, da aufgrund der Feinteiligkeit beispielsweise des oberflächenmodifizierten Zinkoxids und der guten Verteilung eine besonders hohe UV-Absorption erreicht wird.
Ein weiterer Gegenstand der vorliegenden Erfindung ist die Verwendung der erfindungsgemäß oberflächenmodizierten Metalloxide, insbesondere von Zinkoxid als antimikrobieller Wirkstoff. Die Verwendung dieser Partikel ist für diesen Einsatzzweck besonders vorteilhaft, da aufgrund der Feinteiligkeit der Partikel und der daraus resultierenden großen Oberfläche, die antimikrobielle Wirkung stark verbessert ist und andererseits aufgrund der guten Dispergiereigenschaften des Materials das Zinkoxid in fein verteilter Form vorliegt. Somit kann das Zinkoxid problemlos in verschiedenen Darreichungsformen eingesetzt werden wie beispielsweise Cremes, Hautmilch, Lotionen oder Tonics.

Ein weiterer Gegenstand der vorliegenden Erfindung ist ein pharmazeutisches Mittel, das ein erfindungsgemäßes oberflächenmodifiziertes Metalloxid enthält. Dieses pharmazeutische Mittel weist sich dadurch aus, dass aufgrund der Feinteiligkeit der Partikel die pharmazeutische Wirksamkeit stark erhöht ist.

Darüber hinaus besitzt das erfindungsgemäße pharmazeutische Mittel den Vorteil, dass aufgrund der bereits oben beschriebenen guten Langzeitstabilität beispielsweise der Zinkoxiddispersionen auf den Zusatz von Stabilisatoren verzichtet werden kann, die eine Entmischung verhindern. Somit wird zusätzlich die Verträglichkeit des pharmazeutischen Mittels erhöht.

Anhand der folgenden Beispiele soll die Erfindung näher erläutert werden.

Herstellung von oberflächenmodifiziertem Zinkoxid

### Beispiel 1:

1000 ml einer 0,2 M Zn(II)nitrat-Lösung wurden auf 40°C erwärmt und innerhalb von 4 Stunden unter Rühren zu 1000 ml einer ebenfalls auf 40°C erwärmten 0,2 M NaOH-Lösung, die zusätzlich 20 g eines Copolymeren P, bestehend aus 87 mol-% N-Vinylpyrrolidon/10 mol% Laurylacrylat/3 mol% Natriumacrylat enthielt, zudosiert. Das ausgefallene mittels Copolymer P oberflächenmodifizierte Produkt wurde abfiltriert und bei 50°C im Trockenschrank getrocknet.

### Beispiel 2:

1000 ml einer 0,2 M Zn(II)chlorid-Lösung wurden auf 40°C erwärmt und innerhalb von 4 Stunden unter Rühren zu 1000 ml einer ebenfalls auf 40°C erwärmten 0,2 M NaOH-Lösung, die zusätzlich 20 g eines Copolymeren, bestehend aus 65 mol-% N-Vinylpyrrolidon/10 mol-% Laurylacrylat/25 mol-% Natriumacrylat, enthielt, zudosiert. Das ausgefallene mittels Copolymer P oberflächenmodifizierte Produkt wurde abfiltriert und bei 50°C im Trockenschrank getrocknet.

### Beispiel 3:

1000 ml einer 0,4 M NaOH-Lösung, die zusätzlich 4 g eines Copolymeren P, bestehend aus 87 mol-% N-Vinylpyrrofidon/10 mol-% Laurylacrylat/3 mol% Natriumacrylat enthielt, wurden auf 40°C erwärmt und innerhalb von 12 min zu 1000 ml einer ebenfalls auf 40°C erwärmten 0,2 M Zn(II)nitrat-Lösung zudosiert. Das Präzipitat wurde über 4 Stunden bei 40°C gerührt. Das ausgefallene mittels Copolymer P oberflächenmodifizierte Produkt wurde abfiltriert und bei 50°C im Trockenschrank getrocknet.

### Beispiel 4

1000 ml einer 0,4 M NaOH-Lösung, die zusätzlich 4 g eines Copolymeren, bestehend aus 65 mol-% N-Vinylpyrrolidon/10 mol-% Laurylacrylat/25 mol-% Natriumacrylat enthielt, wurden auf 40°C erwärmt und innerhalb von 12 min zu 1000 ml einer ebenfalls auf 40°C erwärmten 0,2 M Zn(II)nitrat-Lösung zudosiert. Das Präzipitat wurde über 4 Stunden bei 40°C gerührt. Das ausgefallene mittels dieses Copolymeren oberflächenmodifizierte Produkt wurde abfiltriert und bei 50°C im Trockenschrank getrocknet.

### Beispiel 5

250 ml einer 0,4 M NaOH-Lösung, die zusätzlich 4 g/l eines Copolymeren bestehend aus 87 mol-% N-Vinylpyrrolidon/10 mol-% Laurylacrylat/3 mol-% Natriumacrylat enthielt, wurden auf 40°C erwämt und innerhalb von ca. 5 Sekunden unter Rühren zu 250 ml einer ebenfalls auf 40°C erwärmten 0,2M Zink(11)acetat-Lösung geschüttet. Das Präzipitat wurde über 2 Stunden bei 40°C gerührt. Anschließend wurde das oberflächenmodifizierte ZnO filtriert und bei Raumtemperatur getrocknet.

### Beispiel 6

500 ml einer 0,4M NaOH-Lösung, die zusätzlich 2g eines Copolymeren P, bestehend aus 80 mol-% N-Vinylcaprolactam/5 mol-% Layrylacrylat/15 mol-% Natriumacrylat enthielt, wurden auf 40°C erwärmt und innerhalb von 6 min zu 500 ml einer ebenfalls auf 40°C erwärmten 0,2 M Zn(11)acetat-Lösung zudosiert. Das Präzipitat wurde über 2 Stunden bei 40°C gerührt. Das ausgefallene mittels Copolymer P oberflächenmodifizierte Produkt wurde abfiltriert und bei Raumtemperatur getrocknet.

### Beispiele für kosmetische Formulierungen

Allgemeine Vorschrift zur Herstellung der erfindungsgemäßen Zubereitungen als Emulsionen

Die jeweiligen Phasen A und C wurden getrennt auf ca. 85°C erwärmt. Anschließend wurde Phase C und das Metalloxid unter Homogenisieren in Phase A eingerührt. Nach kurzem Nachhomogenisieren wurde die Emulsion unter Rühren auf Raumtemperatur abgekühlt und abgefüllt. Alle Mengenangaben sind auf das Gesamtgewicht der Zubereitungen bezogen.

### Beispiel 7:

Emulsion A, enthaltend 3 Gew.-% Uvinul^{®} T150 und 4 Gew.-% Zinkoxid, hergestellt gemäß Beispiel 2

| Phase | % | INCI |
|---|---|---|
| | | |
| A | 8,00 | Dibutyl Adipate |
| | 8,00 | C₁₂-C₁₅ Alkyl Benzoate |
| | 12,00 | Cocoglycerides |
| | 1,00 | Sodium Cetearyl Sulfate |
| | 4,00 | Lauryl Glucoside, Polyglyceryl-2 |
| | 2,00 | Cetearyl Alcohol |
| | 3,00 | Ethylhexyl Triazone (Uvinul^{®} T150) |
| | 1,00 | Tocopheryl Acetate |
| | | |
| B | 4,0 | Zinc Oxide |
| | | |
| C | 3,00 | Glycerin |
| | 0,20 | Allantoin |
| | 0,30 | Xanthan Gum |
| | 0,02 | Triethanolamine |
| | Ad 100 | Aqua dem. |

### Beispiel 8:

Emulsion B, enthaltend 3 Gew.-% Uvinul^{®} T150, 2 Gew.-% Uvinul^{®} A Plus und 4 Gew.-% Zinkoxid, hergestellt nach Beispiel 2

| Phase | % | INCI |
|---|---|---|
| | | |
| A | 8,00 | Dibutyl Adipate |
| | 8,00 | C12-C15 Alkyl Benzoate |
| | 12,00 | Cocoglycerides |
| | 1,00 | Sodium Cetearyl Sulfate |
| | 4,00 | Lauryl Glucoside, Polyglyceryl-2 |
| | 2,00 | Cetearyl Alcohol |
| | 3,00 | Ethylhexyl Triazone (Uvinul^{®} T150) |
| | 1,00 | Tocopheryl Acetate |
| | 2,00 | Diethylamino Hydroxybenzoyl Hexyl Benzoate (Uvinul^{®} A Plus) |
| | | |
| B | 4,0 | Zinc Oxide |
| | | |
| C | 3,00 | Glycerin |
| | 0,20 | Allantoin |
| | 0,30 | Xanthan Gum |
| | 1,50 | Magnesium Aluminium Silicate |
| | ad 100 | Aqua dem. |

### Beispiel 9:

Emulsion A, enthaltend 3 Gew.-% Uvinul^{®} T150 und 4 Gew.-% Zinkoxid, hergestellt gemäß Beispiel 5

| Phase | % | INCI |
|---|---|---|
| | | |
| A | 8,00 | Dibutyl Adipate |
| | 8,00 | C₁₂-C₁₅ Alkyl Benzoate |
| | 12,00 | Cocoglycerides |
| | 1,00 | Sodium Cetearyl Sulfate |
| | 4,00 | Lauryl Glucoside, Polyglyceryl-2 |
| | 2,00 | Cetearyl Alcohol |
| | 3,00 | Ethylhexyl Triazone (Uvinul^{®} T150) |
| | 1,00 | Tocopheryl Acetate |
| | | |
| B | 4,0 | Zinc Oxide |
| | | |
| C | 3,00 | Glycerin |
| | 0,20 | Allantoin |
| | 0,30 | Xanthan Gum |
| | 0,02 | Triethanolamine |
| | Ad 100 | Aqua dem. |

### Beispiel 10:

Emulsion B, enthaltend 3 Gew.-% Uvinul^{®} T150, 2 Gew.-% Uvinul^{®} A Plus und 4 Gew.%. Zinkoxid, hergestellt nach Beispiel 5

| Phase | % | INCI |
|---|---|---|
| | | |
| A | 8,00 | Dibutyl Adipate |
| | 8,00 | C12-C15 Alkyl Benzoate |
| | 12,00 | Cocoglycerides |
| | 1,00 | Sodium Cetearyl Sulfate |
| | 4,00 | Lauryl Glucoside, Polyglyceryl-2 |
| | 2,00 | Cetearyl Alcohol |
| | 3,00 | Ethylhexyl Triazone (Uvinul^{®} T150) |
| | 1,00 | Tocopheryl Acetate |
| | 2,00 | Diethylamino Hydroxybenzoyl Hexyl Benzoate (Uvinul^{®} A Plus) |
| | | |
| B | 4,0 | Zinc Oxide |
| | | |
| C | 3,00 | Glycerin |
| | 0,20 | Allantoin |
| | 0,30 | Xanthan Gum |
| | 1,50 | Magnesium Aluminium Silicate |
| | ad 100 | Aqua dem. |

## Patentansprüche

1. Oberflächenmodifizierte nanopartikuläre Metalloxide, wobei das Metall ausgewählt ist aus der Gruppe, bestehend aus Aluminium, Cer, Eisen, Titan, Zink und Zirkonium, **dadurch gekennzeichnet, dass** die Oberflächenmodifikation eine Beschichtung mit einem Copolymeren P mit einem K-Wert von mindestens 7 bis 130 aus
A) 60 bis 99 mol-% eines N-Vinyllactams, und
B) 1 bis 40 mol-% eines Monomeren, ausgewählt aus der Gruppe der
b₁) C₈-C₃₀-Alkylester von monoethylenisch ungesättigten C₃-C₈-Carbonsäuren
b₂) N-Alkyl- oder N,N-Dialkyl-substituierten Amide der Acrylsäure oder der Methacrylsäure mit C₈-C₁₈-Alkylresten, oder
b₃) der Vinylester von aliphatischen C₈-C₃₀-Carbonsäuren, oder Gemischen dieser Monomeren
C) 0 bis 39 mol% weiterer copolymerisierbarer Monomere
umfasst.

2. Oberflächenmodifizierte Metalloxide nach Anspruch 1, **dadurch gekennzeichnet, dass** die Oberflächenmodifikation eine Beschichtung mit einem Copolymeren umfasst, dessen Komponente A) 60 bis 99 mol% N-Vinylpyrrolidon enthält.

3. Oberflächenmodifizierte Metalloxide nach Anspruch 1 oder 2, **dadurch gekennzeichnet, dass** die Metalloxidpartikel einen mittleren Primärpartikeldurchmesser von 5 bis 10000 nm aufweisen.

4. Metalloxide nach einem der Ansprüche 1 bis 3, **dadurch gekennzeichnet, dass** es sich um oberflächenmodifiziertes Zinkoxid handelt.

5. Verfahren zur Herstellung eines oberflächenmodifizierten nanopartikulären Metalloxids, wobei das Metall ausgewählt ist aus der Gruppe, bestehend aus Aluminium, Cer, Eisen, Titan, Zink und Zirkonium, durch
a. Fällung des Metalloxids aus einer wässrigen Lösung eines seiner Metallsalze, wobei im Falle von Zinkoxid der pH-Wert bei der Fällung im Bereich von 7 bis 11 liegt,
b. Abtrennung des ausgefällten Metalloxids aus der wässrigen Reaktionsmischung und
c. anschließende Trocknung des Metalloxids,
**dadurch gekennzeichnet, dass** die Fällung des Metalloxids im Verfahrensschritt a. in Gegenwart eines Copolymeren P mit einem K-Wert von mindestens 7 bis 130 aus
A) 60 bis 99 mol-% eines N-Vinyllactams, und
B) 1 bis 40 mol-% eines Monomeren, ausgewählt aus der Gruppe der
b₁) C₈-C₃₀-Alkylester von monoethylenisch ungesättigten C₃-C₈-Carbonsäuren
b₂) N-Alkyl- oder N,N-Dialkyl-substituierten Amide der Acrylsäure oder der Methacrylsäure mit C₈-C₁₈-Alkylresten, oder
b₃) der Vinylester von aliphatischen C₈-C₃₀-Carbonsäuren, oder Gemischen dieser Monomeren.
C) 0 bis 39 mol-% weiterer copolymerisierbarer Monomere
erfolgt.

6. Verfahren nach Anspruch 5, **dadurch gekennzeichnet, dass** die Fällung in Gegenwart eines Copolymeren P erfolgt, dessen Komponente A) 60 bis 99 mol-% N-Vinylpyrrolidon enthält.

7. Verfahren nach Anspruch 5 oder 6, **dadurch gekennzeichnet, dass** es sich bei den Metallsalzen um Metallhalogenide, -acetate, -sulfate oder -nitrate handelt.

8. Verfahren nach einem der Ansprüche 5 bis 7, **dadurch gekennzeichnet, dass** die Fällung bei einer Temperatur im Bereich von 20°C bis 100°C erfolgt.

9. Verfahren nach einem der Ansprüche 5 bis 8, **dadurch gekennzeichnet, dass** die Fällung bei einem pH-Wert im Bereich von 3 bis 12 erfolgt.

10. Verfahren nach einem der Ansprüche 5 bis 9 zur Herstellung von oberflächenmodifiziertem nanopartikulären Zinkoxid.

11. Verfahren nach Anspruch 10, **dadurch gekennzeichnet, dass** die Fällung des Zinkoxids im Verfahrensschritt a. aus einer wässrigen Lösung von Zink(II)chlorid, Zink(II)nitrat oder Zink(II)acetat bei einer Temperatur im Bereich von 25 bis 40°C und einem pH-Wert im Bereich von 7 bis 11 in Gegenwart eines Copolymeren P erfolgt.

12. Verwendung von oberflächenmodifizierten nanopartikulären Metalloxiden, definiert gemäß einem der Ansprüche 1 bis 4, zur Herstellung kosmetischer Zubereitungen.

13. Verwendung nach Anspruch 12 zur Herstellung kosmetischer Sonnenschutzmittelzubereitungen.

14. Kosmetische Zubereitungen, enthaltend oberflächenmodifizierte nanopartikuläre Metalloxide, definiert gemäß einem der Ansprüche 1 bis 4.

## Claims

1. A surface-modified nanoparticulate metal oxide,
where the metal is chosen from the group consisting of aluminum, cerium, iron, titanium, zinc and zirconium, wherein the surface modification comprises a coating with a copolymer P having a K value of at least 7 to 130 of
A) 60 to 99 mol% of an N-vinyllactam and
B) 1 to 40 mol% of a monomer chosen from the group of
b₁) C₈-C₃₀-alkyl esters of monoethylentically unsaturated C₃-C₈-carboxylic acids
b₂) N-alkyl- or N,N-dialkyl-substituted amides of acrylic acid or of methacrylic acid with C₈-C₁₈-alkyl radicals, or
b₃) vinyl esters of aliphatic C₈-C₃₀-carboxylic acids, or mixtures of these monomers
C) 0 to 39 mol% of further copolymerizable monomers.

2. The surface-modified metal oxide according to claim 1, wherein the surface modification comprises a coating with a copolymer whose component A) comprises 60 to 99 mol% of N-vinylpyrrolidone

3. The surface-modified metal oxide according to claim 1 or 2, wherein the metal oxide particles have an average primary particle diameter of from 5 to 10 000 nm.

4. The metal oxide according to one of claims 1 to 3,
wherein it is surface-modified zinc oxide.

5. A process for the preparation of a surface-modified nanoparticulate metal oxide, where the metal is chosen from the group consisting of aluminum, cerium, iron, titanium, zinc and zirconium, by
a. precipitation of the metal oxide from an aqueous solution of one of its metal salts, where, in the case of zinc oxide, the pH during the precipitation is in the range from 7 to 11,
b. separating off the precipitated metal oxide from the aqueous reaction mixture and
c. subsequent drying of the metal oxide,
wherein the precipitation of the metal oxide in process step a. takes place in the presence of a copolymer P having a K of at least 7 to 130 of
A) 60 to 99 mol% of an N-vinyllactam and
B) 1 to 40 mol% of a monomer chosen from the group of
b₁) C₈-C₃₀-alkyl esters of monoethylenically unsaturated C₃-C₈-carboxylic acids
b₂) N-alkyl- or N,N-dialkyl-substituted amides of acrylic acid or of methacrylic acid with C₈-C₁₈-alkyl radicals, or
b₃) vinyl esters of aliphatic C₈-C₃₀-carboxylic acids, or mixtures of these monomers.
C) 0 to 39 mol% of further copolymerizable monomers

6. The process according to claim 5, wherein the precipitation takes place in the presence of a copolymer P whose component A) comprises 60 to 99 mol% of N-vinylpyrrolidone.

7. The process according to claim 5 or 6, wherein the metal salts are metal halides, acetates, sulfates or nitrates.

8. The process according to one of claims 5 to 7, wherein the precipitation takes place at a temperature in the range from 20°C to 100°C.

9. The process according to one of claims 5 to 8,
wherein the precipitation takes place at a pH in the range from 3 to 12.

10. The process according to one of claims 5 to 9 for the preparation of surface-modified nanoparticulate zinc oxide.

11. The process according to claim 10, wherein the precipitation of the zinc oxide in process step a. takes place from an aqueous solution of zinc (II) chloride, zinc(II) nitrate or zinc(II) acetate at a temperature in the range from 25 to 40°C and a pH in the range from 7. to 11 in the presence of a copolymer P.

12. The use of surface-modified nanoparticulate metal oxides, defined according to one of claims 1 to 4, for producing cosmetic preparations.

13. The use according to claim 12 for producing cosmetic sunscreen preparations.

14. A cosmetic preparation comprising surface-modified nanoparticulate metal oxides defined according to one of claims 1 to 4.

## Revendications

1. Oxydes métalliques nanoparticulaires modifiés en surface, le métal étant choisi parmi le groupe composé de l'aluminium, du cérium, du fer, du titane, du zinc et du zirconium, **caractérisés en ce que** la modification des surfaces comprend un revêtement par un copolymère P d'une valeur K d'au moins 7 à 130 constitué de :
A) 60 à 99 % en mole d'un N-vinyllactame,
B) 1 à 40 % en mole d'un monomère choisi parmi le groupe des :
b₁) alkylesters en C₈-C₃₀ d'acides carboxyliques en C₃-C₈ monoéthyléniquement insaturés,
b₂) amides substitués par N-alkyle ou N,N-dialkyle de l'acide acrylique ou de l'acide méthacrylique avec des radicaux alkyle en C₈-C₁₈, ou
b₃) vinylesters d'acides carboxyliques en C₈-C₃₀ aliphatiques ou des mélanges de ces monomères, et
C) 0 à 39 % en mole d'autres monomères copolymérisables.

2. Oxydes métalliques modifiés en surface selon la revendication 1, **caractérisés en ce que** la modification des surfaces comprend le revêtement avec un copolymère dont le composant A contient de 60 à 99 % en mole de N-vinylpyrrolidone.

3. Oxydes métalliques modifiés en surface selon la revendication 1 ou 2, **caractérisés en ce que** les particules d'oxydes métalliques présentent un diamètre moyen de particules primaires de 5 à 10 000 nm.

4. Oxydes métalliques selon l'une des revendications 1 à 3, **caractérisés en ce qu'**il s'agit d'oxyde de zinc modifié en surface.

5. Procédé de fabrication d'un oxyde métallique nanoparticulaire modifié en surface, le métal étant choisi parmi le groupe composé de l'aluminium, du cérium, du fer, du titane, du zinc et du zirconium, ledit procédé consistant à :
a. précipiter l'oxyde métallique à partir d'une solution aqueuse d'un de ses sels métalliques, dans le cas de l'oxyde de zinc, le pH lors de la précipitation se situant dans l'intervalle compris entre 7 et 11,
b. séparer l'oxyde métallique précipité du mélange réactionnel aqueux, et
c. sécher ensuite l'oxyde métallique,
**caractérisé en ce que** la précipitation de l'oxyde métallique à l'étape de procédé a. a lieu en présence d'un copolymère P d'une valeur K d'au moins 7 à 130 constitué de :
A) 60 à 99 % en mole d'un N-vinyllactame,
B) 1 à 40 % en mole d'un monomère choisi parmi le groupe des :
b₁) alkylesters en C₈-C₃₀ d'acides carboxyliques en C₃-C₈ monoéthyléniquement insaturés,
b₂) amides substitués par N-alkyle ou N,N-dialkyle de l'acide acrylique ou de l'acide méthacrylique avec des radicaux alkyle en C₈-C₁₈, ou
b₃) vinylesters d'acides carboxyliques en C₈-C₃₀ aliphatiques ou des mélanges de ces monomères, et
C) 0 à 39 % en mole d'autres monomères copolymérisables.

6. Procédé selon la revendication 5, **caractérisé en ce que** la précipitation a lieu en présence d'un copolymère P dont le composant A contient de 60 à 99 % en mole de N-vinylpyrrolidone.

7. Procédé selon la revendication 5 ou 6, **caractérisé en ce que** pour les sels métalliques, il s'agit d'halogénures, d'acétates, de sulfates ou de nitrates métalliques.

8. Procédé selon l'une des revendications 5 à 7,
**caractérisé en ce que** la précipitation a lieu à une température comprise dans l'intervalle entre 20 °C et 100 °C.

9. Procédé selon l'une des revendications 5 à 8,
**caractérisé en ce que** la précipitation a lieu à un pH dans l'intervalle compris entre 3 et 12.

10. Procédé selon l'une des revendications 5 à 9 de fabrication d'oxyde de zinc nanoparticulaire modifié en surface.

11. Procédé selon la revendication 10, **caractérisé en ce que** la précipitation de l'oxyde de zinc à l'étape de procédé a. a lieu à partir d'une solution aqueuse de chlorure de zinc (II), de nitrate de zinc(II) ou d'acétate de zinc(II) à une température dans l'intervalle compris entre 25 et 40 °C et à un pH dans l'intervalle compris entre 7 et 11 en présence d'un copolymère P.

12. Utilisation d'oxydes métalliques nanoparticulaires modifiés en surface définie selon l'une des revendications 1 à 4, pour la fabrication de préparations cosmétiques.

13. Utilisation selon la revendication 12 pour la fabrication de préparations cosmétiques destinées à des produits solaires.

14. Préparations cosmétiques contenant des oxydes métalliques nanoparticulaires modifiés en surface, définies selon l'une des revendications 1 à 4.
